# EUROPÄISCHE PATENTSCHRIFT

(11) **EP 3 160 550 B1**
(45) Veröffentlichungstag und Bekanntmachung des Hinweises auf die Patenterteilung: **04.10.2023**
(21) Anmeldenummer: 15739529.4
(22) Anmeldetag: 24.06.2015
(51) Int. Cl.: A61M 5/30

(54) **NADELLOSE INJEKTIONSVORRICHTUNG MIT DOPPELMEMBRAN**
NEEDLE-FREE INJECTION DEVICE WITH DOUBLE MEMBRANE
DISPOSITIF D'INJECTION SANS AIGUILLE AVEC DOUBLE MEMBRANE

(30) Priorität: 24.06.2014 EP 14173786
(43) Veröffentlichungstag der Anmeldung: 03.05.2017
(73) Patentinhaber: Lell, Peter, 85368 Moosburg (DE)
(72) Erfinder: LELL, Peter, 85368 Moosburg (DE); ANAMUR, Cihad, 67059 Ludwigshafen (DE); FELLNER, Christian, 85304 Ilmmünster (DE); WINTER, Gerhard, 82377 Penzberg (DE); ENGERT, Julia, 80993 München (DE)
(74) Vertreter: Simandi, Claus
(86) Internationale Anmeldenummer: PCT/EP2015/064304
(87) Internationale Veröffentlichungsnummer: WO 2015/197724

(56) Entgegenhaltungen:
- WO-A1-01/41839
- WO-A1-2004/071558
- WO-A2-98/31409
- US-A- 6 010 478
- US-A1- 2007 038 175

## Beschreibung

Die Erfindung betrifft eine nadellose Injektionsvorrichtung bzw. eine Vorrichtung zur nadellosen Injektion einer Substanz in ein Gewebe oder Körper, mit welcher vorzugsweise ein Stoff, insbesondere Wirkstoff mittels hohem Druck injiziert werden kann. Eine erfolgreiche Injektion besteht darin, dass genügend Substanz durch die Gewebe-, oder Körperbarriere, insbesondere solche wie die Haut, in den Körper eingebracht wird. Das Grundprinzip, einen Stoff nadellos mittels hohem Druck zu injizieren, ist seit geraumer Zeit bekannt (siehe z. B. US 3,308,818).

Es sind verschiedene Typen von nadellosen Injektionsvorrichtungen im Stand der Technik beschrieben, wie z. B. nicht abschließend eine Zylinder-Kolben-Einheit umfassend ein vorgespanntes Federelement (DE 10 2007 004 211 A1) oder mittels sogenannter Injektionspatronen (WO 98/31409) als auch mittels einer Gaspatrone zur Beaufschlagung eines Kolbens (US 5,399,163).

Weiterhin können nadellose Injektionsvorrichtungen aus einem pyrotechnischen Antrieb bestehen, der beispielsweise zur Betätigung eines Kolbens genutzt wird und auf diese Weise das Auspressen eines Wirkstoffes aus einer Kanüle erzeugt (EP 1 557 190 A1).

Eine weitere Ausführungsform einer nadellosen Injektionsvorrichtung besteht in einem pyrotechnischen Ansatz, wobei in einer Kammer (auch Brennkammer genannt) eine Explosion erzeugt wird, wobei die freigesetzte Energie zur Impulsübertragung auf eine Membran genutzt wird, wodurch eine membranständige adsorbierte Substanz unter Ablösung von der Membran in Richtung des Gewebes hinreichend beschleunigt wird.

Eine solche gattungsmäßige Vorrichtung ist in WO 2004/071558 A1 offenbart. Es gilt jedoch eine solche gattungsmäßige Ausführungsform zu verbessern. Insbesondere ist gemäß der Lehre von WO 2004/071558 nachteilig, dass die vorgesehene Membran platzen kann und folglich nicht ausgeschlossen ist, dass Teile der Membran als auch die Abgase aus der Brennkammer / Verbrennungsraum das Gewebe unter Schädigung des Gewebes bzw. Körpers erreichen.

Von daher nimmt die vorliegende Erfindung Bezug auf eine gattungsmäßige Ausführungsform gemäß WO 2004/071558, jedoch mit der objektiven Aufgabenstellung, das Gewebe oder einen Körper von Membranteilen und Abgasen freizuhalten.

Daher betrifft die vorliegende Erfindung eine nadellose Injektionsvorrichtung umfassend eine (Brenn-)Kammer (11) enthaltend ein pyrotechnisches Material, wobei die Kammer (11) an einer Austrittsöffnung eine fixierte Doppelmembran (13, 15) aufweist, wobei die hautseitige Membran (15) der Doppelmembran mit einer Substanzauftragung (25) zur Hautseite der hautseitigen Membran (15) versehen ist, wobei die brennkammerseitige Membran (13) und die hautseitige Membran (15) zur Kammer (11) hin ganz oder teilweise ausgewölbt ist.

Die brennkammerseitige Membran (13) mündet zur Brennkammer (11) und dessen Verbrennungsraum (24).

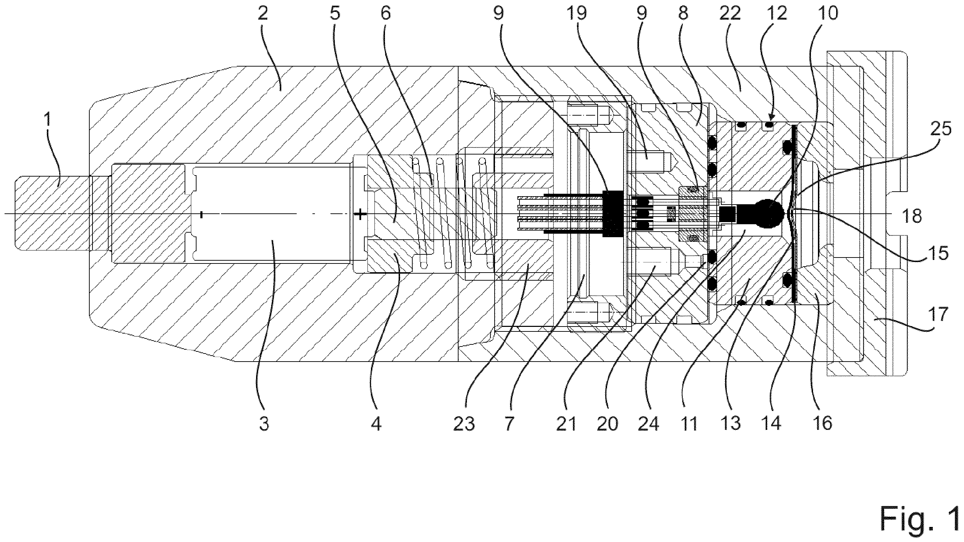

Ein grundlegender und gattungsgemäßer Aufbau ist in Figur 1 samt Legende gezeigt, wobei für die erfindungsgemäße Funktion der Injektionsvorrichtung das Gehäuse (22), sowie die Teile (7) bis (16) zu berücksichtigen sind. Die Teile (7) bis (16) können hierbei jederzeit in ein anders geformtes Gehäuse (22) eingebaut und ein Detonator, insbesondere EED (10), auch anders mit Energie versorgt werden, ohne die Funktion der Teile bzw. der Vorrichtung zu verändern. Besondere Ausführungsformen werden näher beschrieben.

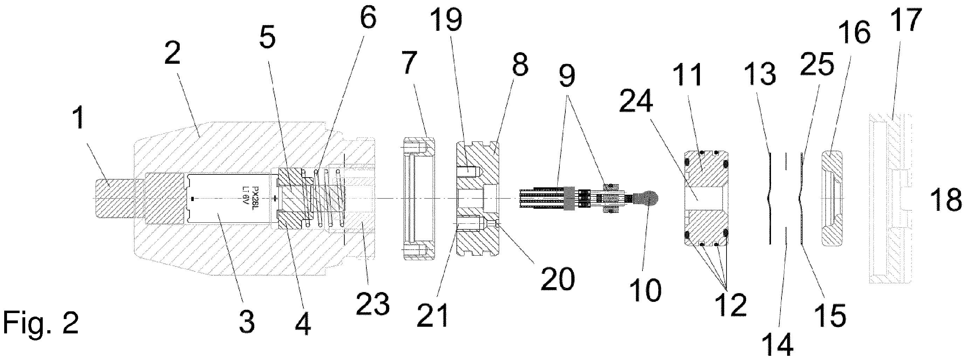

Die Geometrie der einzelnen Teile in Figur 1 können je nach Anforderung geändert oder teilweise integral zusammengefasst werden. Beispielsweise können Teile (7) bis (9) zu einem Teil integral zusammengefasst werden, oder Teil (7) kann entfallen, falls Teil (8) beispielsweise ein Außengewinde aufweist und in das Gehäuse (22) eingeschraubt wird - oder beispielsweise per Sprengring im Gehäuse (22) gehalten wird - oder ein Einlegeteil in der Spritzform des Gehäuses (22) ist (siehe ebenfalls Figur 2). Ebenfalls können die Teile (1), sowie die Teile (4) bis (6) und (23) durch die Verwendung eines herkömmlichen Tasters zur elektrischen Kontaktierung des Detonators (EED) (10) mit der Batterie (3) entfallen.

Im Rahmen dieser Erfindung wird unter "pyrotechnisches Material" ein jedes Material verstanden, welches mit einer Aktivierungsenergie zur Explosion gebracht werden kann. Dies können z.B. feste oder gasförmige Stoffe, wie Azide, Tetrazen, etc. oder anderen dem Fachmann bekannte pyrotechnische Materialien sein. Erfindungsgemäß soll die Explosionsenergie eine hinreichende Impulsübertragung auf die erfindungsgemäße fixierte Doppelmembran (13, 15) erlauben, so dass die aufgetragene Substanz (25) abgelöst, beschleunigt und auf hohe Geschwindigkeiten gebracht werden kann.

Das pyrotechnische Material ist erfindungsgemäß in einer Kammer (11) enthalten und zwar im Verbrennungsraum (24), wobei die erforderliche Aktivierungsenergie zur Erzeugung einer Explosionsenergie vorzugsweise mittels eines Detonators (10), insbesondere mittels eines Minidetonators (EED) (10) erfolgt.

Die erforderliche Aktivierungsenergie zur Explosionsauslösung in der Kammer (11) kann daher mittels einer Aktivierungseinheit erfolgen, insbesondere nicht abschließend einem Detonator (10), Anzünder, Zündstift oder Anzündstück und zwar über einen Auslösungsmechanismus mittels Reibung, Stoßen oder geeigneter Stromversorgung. Eine geeignete Aktivierungseinheit ist beispielsweise in Figur 1 dargestellt. Mittels eines Auslösers oder Tasters (1) wird die Batterie (3) gegen eine Kontaktfeder (6) und gegen einen Kontaktstift (5) gedrückt, dadurch der Stromkreis über die Anschlüsse (9) und das EED (10) geschlossen und infolge die Zündung des EEDs (10) herbeigeführt. Alternative Auslösungsmechanismen sind dem Fachmann bekannt (Reibdraht, Schlagdraht, Knackfrosch auf stoßempfindliche Anzündmischung schlagend etc.).

In einer weiteren bevorzugten Ausführungsform ist die besagte Aktivierungseinheit (1, 2, 3, 4, 5, 6, 7, 8, 9, 10) in axialer Richtung zur Kammer (11) samt Verbrennungsraum (24) ausgerichtet und der fixierten Doppelmembran (13, 15) gegenüberliegend.

Beispielsweise bei 20 bis 80 µm großen Zuckerpartikeln als aufgetragene Substanz (25) sind erfindungsgemäß Geschwindigkeiten von ca. 600 m/sec oder mehr zu erreichen. Größere Teilchen dringen wegen Ihres Impulses und des anwachsenden Verhältnisses von Dichte zum Radius deutlich einfacher in das Gewebe oder in den Körper ein, während kleinere Partikel hingegen eine deutlich höhere Geschwindigkeit als die genannten 600 m/sec benötigen.

Falls kleinere Geschwindigkeiten der durch die Doppelmembran beschleunigten Teilchen bis ca. 400 m/sec ausreichen, kann beispielsweise anstelle des Detonators 10 auch ein herkömmlicher Anzünder mit oder ohne zusätzlicher Aufladung verwendet werden.

Im Rahmen dieser Erfindung wird unter "Impuls(übertragung)" auch synonym eine Kraft-, Druckübertragung oder Beaufschlagung verstanden, welche jedenfalls ausreichend ist, die auf der hautseitigen Membran (15) der Doppelmembran (13, 15) aufgetragenen Substanz(en) (25) abzulösen und auf die gewünschten hohen Geschwindigkeiten zu beschleunigen, wodurch sie zunächst frei in Richtung des Gewebes bzw. Haut (18) oder Körper fliegen, um dort einzuschlagen, die Gewebe- oder Körperbarriere zu durchbrechen und in die gewünschte Tiefe des Gewebes, insbesondere Haut oder Körpers einzudringen.

Im Rahmen dieser Erfindung bedeutet "Haut" eine Gewebebarriere eines Menschen, Säugetier oder Tier. Die Haut übt als das größte Organ des Menschen zahlreiche lebenswichtige Funktionen aus, insbesondere die Epidermis, dient als Barriereorgan. Diese Barrierefunktion wird unter anderem von Hautlipiden aufrechterhalten. Diese epidermalen Lipide wie z.B. Glycosphingolipide, Ceramide, Sterine und Sterinester, Fettsäuren, Triglyceride, n-Alkane oder verschiedenen polaren Lipiden werden im Keratinisierungsprozess freigesetzt. Durch die erfindungsgemäße nadellose oder nadelfreie Applikation können infolge dessen die Stoffe in den Körper lokal eintreten und ggfs. in die Blutbahnen münden.

In einer weiteren Ausführungsform kann der Verbrennungsraum (24) in der Kammer (11) vorzugsweise mit einer viskosen zähflüssigen Masse versehen sein, wie Fett, Öle und dergleichen, zwecks Verkleinerung des Leerraumes im Verbrennungsraum (24) und dadurch eine Erhöhung des Druckanstieges bei kleiner Aufladung des Detonators (EED) (10) bewirken.

Die Kammer (11) weist vorzugsweise O-Ringe zur Abdichtung (12) auf. Weiterhin kann die Kammer (11) eine Entlüftung (20) samt zugehöriger Abdeckung (21) aufweisen.

Wie oben bereits ausgeführt, offenbart die gattungsmäßige zugeordnete WO 2004/071558 zwar eine Membran, jedoch keine erfindungsgemäße fixierte Doppelmembran (13, 15). Insbesondere die nachstehenden Ausführungsformen erlauben eine effektive Impulsübertragung auf die fixierte Doppelmembran im Sinne dieser Erfindung.

In einer bevorzugten Ausführungsform besteht die erfindungsgemäße fixierte Doppelmembran (13, 15), vorzugsweise sowohl die hautseitige Membran (15) als auch die brennkammerseitge Membran (13) aus Metall oder einem Material entsprechender Härte und Duktilität, solche wie Stahl, Kunststoffen, besonders bevorzugt jedoch Titan oder Titanblech. Insbesondere Titan weist eine vorteilhafte hohe Ziehgrenze bei sehr guter Beschleunigbarkeit durch seine geringere spezifische Dichte gegenüber Stahl und Kunststoffen auf, wodurch bei relativ kleinen Drücken in der Brennkammer eine deutlich höhere Geschwindigkeit der ausbeulenden Membran erreicht wird. Hier maßgeblich ist die Beschleunigungskennzahl BK=sigma 0,2/ro. Ferner sind die Membranen (13, 15) bündig oder geschlossen, d.h. ohne Löcher, Aussparungen etc.

Gegenüber einer Einfachmembran vereint hierbei die fixierte Doppelmembran eine höhere Betriebssicherheit bei Materialfehlern oder Überlastungen (falls eine Membran reißt, dringt immer noch kein Abgas nach außen) mit einer deutlich besseren Verformbarkeit, was die Energieverluste beim Verformen der Doppelmembran minimieren lässt (d.h. beispielsweise statt einer 1 mm starken Einfachmembran werden zwei Membranen mit jeweils 0,5 mm übereinandergelegt, um eine Doppelmembran auszubilden. Ebenfalls andere Stärkeverhältnisse sind möglich, wie beispielsweise 0,6 mm Richtung Brennkammer und 0,4 mm Richtung Haut.

Der Begriff "fixierte Doppelmembran" (13, 15) bedeutet, dass beide Membranen - die Doppelmembran - mit einer Stützscheibe (16), insbesondere an die Kammer (11), angebracht / fixiert sind und folglich nicht beweglich also arretiert sind und vorzugsweise die fixierte Doppelmembran bzw. die brennkammerseitige Membran (13) unmittelbar in die Kammer (11) bzw. Verbrennungsraum (24) mündet (siehe Fig. 1). Weiterhin ist bevorzugt, dass die nach innen gerichtete brennkammerseitige Membran (13) eine Dicke von 0,1 mm bis 0,6 mm aufweist, vorzugsweise 0,2 mm bis 0,6 mm, besonders bevorzugt 0,5 mm. Die nach außen gerichtete hautseitige Membran (15) weist vorzugsweise ebenfalls eine Dicke von 0,1 mm bis 0,6 mm auf, vorzugsweise 0,2 mm bis 0,6 mm besonders bevorzugt 0,5 mm. Die Membranen (13, 15) können sich ganz oder teilweise berühren.

In einer weiteren bevorzugten Ausführungsform weist jedoch die brennkammerseitige Membran (13) zur hautseitigen Membran (15) einen Abstand auf und zwar vorzugsweise von 0,2 mm bis 1,5 mm, besonders bevorzugt 1 mm. Der erforderliche Abstand kann mittels eines Abstandhalters (14) zwischen den beiden Membranen ausgelegt werden. Diese Maßnahme bewirkt eine effiziente Verhinderung des Platzens oder Bersten der hautseitigen Membran.

Erfindungsgemäß ist die Doppelmembran (13, 15) zur Kammer (11) und in dessen Verbrennungsraum (24) hin ganz oder teilweise ausgewölbt, ganzflächig oder vorzugsweise im zentralen radialen Bereich um einen Kreismittelpunkt. Eine solche jeweils konkave Wölbung der jeweiligen Membranen aus Sicht der Brennkammer kann der Fachmann mittels bekannten Methoden erreichen. Die besagte erfindungsgemäße Wölbung der Doppelmembran (13, 15) erlaubt zudem eine vorteilhafte erhöhte Geschwindigkeit von 10 bis 15 % samt Fokussierung und Beschleunigung der aufgetragenen Substanzen (25) in Richtung Haut (18) - vermutlich durch Stoßwellenfokussierung.

In einer weiteren bevorzugten Ausführungsform weisen die beiden Membranen (13, 15) Stützscheiben (16) auf. Die Stützscheiben sind vorzugsweise aus Kunststoff, Messing etc., welche zur energetischen Aufnahme besonders geeignet ist. Darüber hinaus ist die Stützscheibe energetisch angepasst, vorzugsweise mit einer Verjüngung zur Haut (18) hin. Gleichzeitig kann die Stützscheibe (16) an seiner Innenkontur mit einem weichen Kunststoff dünn überzogen sein, z.B. mit Polyethylen, um das Auslösegeräusch der Vorrichtung vorteilhaft deutlich zu reduzieren - es wird beispielsweise das knallartige Geräusch vermieden, welches entsteht, wenn eine Metallmembran auf eine metallene Stützscheibe aufschlägt.

Die Stützscheibe hat zudem auch die Aufgabe, die Membranen nach deren Druckbeaufschlagung energetisch zu verzögern und den nach der Auslösung in der Brennkammer noch wirkenden hohen Druck langdauernd abzufangen und die Membranen zu stützen.

Die vorgenannten Ausführungsformen erlauben vorteilhaft das sichere Nicht-Platzen oder Nicht-Bersten mindestens der äußeren (hautseitigen) Membran der Doppelmembran. Weiterhin wird vorteilhaft erreicht, dass die aufgebrachten Substanzen eine optimierte Impulsübertragung bzw. Beschleunigung auf sehr hohe Geschwindigkeiten bis zu 800m/sec erfahren.

Im Rahmen dieser Erfindung bedeutet "aufgebrachte Substanzen (25)", solche, die zumindest einen Stoff enthalten und beispielsweise mit Ölen u.a. an der äußeren (hautseitigen) Membran (15) fixiert sind oder durch Adhäsion anhaften, wobei die aufgebrachten Substanzen (25) zur Hautseite der hautseitigen Membran (15) aufgebracht ist. Beispielsweise können die aufgebrachten Substanzen Mittel enthalten, die ein Trocknen des Wirkstoffes auf der besagten Membran erlauben. Insbesondere sind zusätzliche Haftvermittler oder Additive geeignet und beizufügen, wie Öle oder andere vorzugsweise pharmazeutisch geeignete Hilfs- und Zusatzstoffe.

Der Begriff "Substanzen" umfasst sämtliche Mittel und Stoffe, die zur zweckmäßigen Auftragung auf die Membran geeignet sind (z.B. Pulver, Partikel etc.), einschließlich ein oder mehrerer Wirkstoffe (z.B. Arzneimittel).

In einer weiteren Ausführungsform kann die Injektionsvorrichtung einen Aufsatz (17) enthalten, welcher ergonomisch an das aufsetzende Gewebe oder Haut (18) angepasst ist. Der Aufsatz (17) hat die Aufgabe, einmal die nach der Auslösung vorgewölbte Doppelmembran sicher von der Oberfläche der anliegenden Haut abzuhalten.

Ferner können in den Aufsatz (17) eingebrachte Bohrungen oder Fräsungen die mit der Beschleunigung der Doppelmembran gleichzeitig mitbeschleunigte Luft parallel zur Oberfläche abströmen lassen und ein hydraulisches Ankoppeln der Haut an die zentrale Bohrung über der Doppelmembran verhindern, welche die anliegende Haut unzulässig mit Gasdruck belasten würde.

Weiterhin ist die Verwendung oder Anwendung von ein oder mehreren Stoffen, insbesondere Wirkstoffen, insbesondere eines Arzneimittels zur nadelfreien Applikation mittels der erfindungsgemäßen Injektionsvorrichtung nach einer der vorstehenden Ausführungsformen möglich.

Weiterhin ist die Verwendung oder Anwendung von Stoffen, insbesondere Stoffen mit beliebiger Funktion und Eignung, solche wie Farb- oder Schmierstoffen oder Materialien mit anderen besonderen Eigenschaften zur nadellosen Einbringung in Oberflächen oder Körper technischer Gegenstände mittels der erfindungsgemäßen Injektionsvorrichtung nach einer der vorstehenden Ausführungsformen möglich.

Daher betrifft die Erfindung eine Injektionsvorrichtung nach einer der vorstehenden Ausführungsformen zur Verwendung eines Stoffes oder eines Wirkstoffes, insbesondere Arzneimittel, zur nadelfreien Applikation.

Die folgenden Beispiele und Figuren sollen die Erfindung näher erläutern, ohne jedoch diese zu beschränken.
Figur 1 zeigt einen Querschnitt der erfindungsgemäßen Injektionsvorrichtung.
Figur 2 zeigt die Bestandteile zur Herstellung der erfindungsgemäßen Injektionsvorrichtung deutlicher.

Legende:
1 Auslöser, Taster
2 Auslösergehäuse
3 Batterie
4 Federkappe
5 Kontaktstift
6 Kontaktfeder
7 Nutmutter
8 Halter für Glasdurchführung
9 Glasdurchführung mit Anschlüsse
10 Minidetonator oder Anzündstück, EED
11 Brennkammer oder Kammer
12 O-Ring-Abdichtung der Brennkammer (kann bei einigen Ausführungsformen der Vorrichtung auch entfallen)
13 Brennkammerseitige Membran, fixiert zur Kammer (11)
14 Abstandshalter, übernimmt auch Abdichtfunktionen
15 Hautseitige Membran, fixiert zu Membran (13) und Abstandshalter (14)
16 Stützscheibe
17 Aufsatz
18 Haut oder die zu penetrierende Oberfläche (liegt auf 17 an, nicht gezeichnet)
19 Gewinde für Masseanschlussschraube (kann bei einigen Ausführungsformen der Vorrichtung auch entfallen)
20 Brennkammerentlüftung
21 Abdeckung, Entlüftung
22 Gehäuse für die Teile 7-21
23 Führung für Kontaktstift
24 Verbrennungsraum
25 Substanzauftragung

## Patentansprüche

1. Nadellose Injektionsvorrichtung umfassend eine Kammer (11) enthaltend ein pyrotechnisches Material, wobei die Kammer (11) an einer Austrittsöffnung eine fixierte Doppelmembran (13, 15) aufweist, wobei die fixierte Doppelmembran aus einer hautseitigen Membran (15) sowie einer brennkammerseitigen Membran (13) besteht, wobei die hautseitige Membran (15) der Doppelmembran (13, 15) mit einer Substanzauftragung (25) zur Hautseite der hautseitigen Membran (15) versehen ist, **dadurch gekennzeichnet, dass** die brennkammerseitige Membran (13) und die hautseitige Membran (15) zur Kammer (11) hin ganz oder teilweise ausgewölbt ist.

2. Nadellose Injektionsvorrichtung nach Anspruch 1, wobei die Doppelmembran (13, 15) mittels mindestens einer Stützscheibe (16) angebracht / fixiert ist.

3. Nadellose Injektionsvorrichtung nach Anspruch 2, wobei die Doppelmembran (13, 15) mittels mindestens einer Stützscheibe (16) an die Kammer (11) angebracht / fixiert ist.

4. Nadellose Injektionsvorrichtung nach einem der vorstehenden Ansprüche, wobei die brennkammerseitige Membran (13) eine Dicke von 0,1 mm bis 0,6 mm aufweist, und / oder die hautseitige Membran (15) eine Dicke von 0,1 mm bis 0,6 mm aufweist.

5. Nadellose Injektionsvorrichtung nach einem der vorstehenden Ansprüche, wobei die fixierte Doppelmembran (13, 15) aus Metall, Stahl, Titan oder Titanblech besteht.

6. Nadellose Injektionsvorrichtung nach einem der vorstehenden Ansprüche, wobei die brennkammerseitige Membran (13) zur hautseitigen Membran (15) einen Abstand von 0,2 mm bis 1,5 mm aufweist.

7. Nadellose Injektionsvorrichtung nach einem der vorstehenden Ansprüche, wobei zwischen der brennkammerseitigen Membran (13) und der hautseitigen Membran (15) ein Abstandshalter (14) vorgesehen ist.

8. Nadellose Injektionsvorrichtung nach einem der vorstehenden Ansprüche, wobei eine Aktivierungseinheit (1, 2, 3, 4, 5, 6, 7, 8, 9, 10) in axialer Richtung zur Kammer (11) ausgerichtet und der fixierten Doppelmembran (13, 15) gegenüberliegend ist.

9. Nadellose Injektionsvorrichtung nach einem der vorstehenden Ansprüche enthaltend einen Aufsatz (17) mit eingebrachten Bohrungen oder Fräsungen.

10. Nadellose Injektionsvorrichtung nach Anspruch 1, wobei die Membranen (13, 15) sich ganz oder teilweise berühren.

## Claims

1. A needleless injection device comprising a chamber (11) containing a pyrotechnic material, the chamber (11) having a fixed double membrane (13, 15) at a discharge opening, the fixed double membrane consisting of a skin-side membrane (15) and a combustion-chamber-side membrane (13) wherein the skin-side membrane (15) of the double membrane (13, 15) is provided with a substance application (25) to the skin side of the skin-side membrane (15), **characterized in that** the combustion-chamber-side membrane (13) and the skin-side membrane (15) are wholly or partially curved towards the chamber (11).

2. A needleless injection device according to claim 1, wherein the double membrane (13, 15) is attached / fixed by means of at least one support disc (16).

3. A needleless injection device according to claim 2, wherein the double membrane (13, 15) is attached / fixed to the chamber (11) by means of at least one support disc (16).

4. A needleless injection device according to any one of the preceding claims, wherein the combustion chamber side membrane (13) has a thickness of 0.1 mm to 0.6 mm, and/or the skin side membrane (15) has a thickness of 0.1 mm to 0.6 mm.

5. A needleless injection device according to any one of the preceding claims, wherein the fixed double membrane (13, 15) is made of metal, steel, titanium or titanium sheet.

6. A needleless injection device according to any one of the preceding claims, wherein the combustion chamber-side membrane (13) is at a distance of 0.2 mm to 1.5 mm from the skin-side membrane (15).

7. A needleless injection device according to one of the preceding claims, wherein a spacer (14) is provided between the combustion chamber-side membrane (13) and the skin-side membrane (15).

8. A needleless injection device according to any one of the preceding claims, wherein an activation unit (1, 2, 3, 4, 5, 6, 7, 8, 9, 10) is aligned in the axial direction with respect to the chamber (11) and is opposite the fixed double membrane (13, 15).

9. A needleless injection device according to one of the preceding claims comprising an attachment (17) with inserted bores or milled holes.

10. A needleless injection device of claim 1, wherein the membranes (13, 15) are in full or partial contact.

## Revendications

1. Dispositif d'injection sans aiguille comprenant une chambre (11) contenant une matière pyrotechnique, la chambre (11) ayant une double membrane fixe (13, 15) au niveau d'une ouverture de décharge, la double membrane fixe consistant en une membrane côté peau (15) et une membrane côté chambre de combustion (13) dans laquelle la membrane côté peau (15) de la double membrane (13, 15) est pourvue d'une application de substance (25) sur le côté peau de la membrane côté peau (15), **caractérisée par le fait que** la membrane côté chambre de combustion (13) et la membrane côté peau (15) sont entièrement ou partiellement incurvées vers la chambre (11).

2. Dispositif d'inj ection sans aiguille selon la revendication 1, dans lequel la double membrane (13, 15) est attachée/fixée au moyen d'au moins un disque de support (16).

3. Dispositif d'inj ection sans aiguille selon la revendication 2, dans lequel la double membrane (13, 15) est attachée/fixée à la chambre (11) au moyen d'au moins un disque de support (16).

4. Dispositif d'injection sans aiguille selon l'une quelconque des revendications précédentes, dans lequel la membrane latérale de la chambre de combustion (13) a une épaisseur comprise entre 0,1 mm et 0,6 mm, et/ou la membrane latérale de la peau (15) a une épaisseur comprise entre 0,1 mm et 0,6 mm.

5. Dispositif d'injection sans aiguille selon l'une quelconque des revendications précédentes, dans lequel la double membrane fixe (13, 15) est en métal, en acier, en titane ou en feuille de titane.

6. Dispositif d'injection sans aiguille selon l'une des revendications précédentes, dans lequel la membrane côté chambre de combustion (13) est à une distance de 0,2 mm à 1,5 mm de la membrane côté peau (15).

7. Dispositif d'injection sans aiguille selon l'une des revendications précédentes, dans lequel une entretoise (14) est prévue entre la membrane côté chambre de combustion (13) et la membrane côté peau (15).

8. Dispositif d'injection sans aiguille selon l'une quelconque des revendications précédentes, dans lequel une unité d'activation (1, 2, 3, 4, 5, 6, 7, 8, 9, 10) est alignée dans la direction axiale par rapport à la chambre (11) et se trouve en face de la double membrane fixe (13, 15).

9. Dispositif d'injection sans aiguille selon l'une des revendications précédentes, comprenant un embout (17) avec des alésages insérés ou des trous fraisés.

10. Dispositif d'injection sans aiguille de la revendication 1, dans lequel les membranes (13, 15) sont en contact total ou partiel.
